(19) Europäisches Patentamt
European Patent Office
Office européen des brevets

(11) Veröffentlichungsnummer: **0 026 323**
**B1**

(12) # EUROPÄISCHE PATENTSCHRIFT

(45) Veröffentlichungstag der Patentschrift: **25.08.82**

(51) Int. Cl.³: **C 07 C 49/553**, C 07 C 45/61, A 61 K 7/46

(21) Anmeldenummer: **80105033.7**

(22) Anmeldetag: **25.08.80**

(54) **4(5)-Acetyl-9,9-dimethyltricyclo-(4.4.0.1(8,10))-undec-1-en, dessen Herstellung und Verwendung als Riechstoff, sowie dieses enthaltende Riechstoffkompositionen.**

(30) Priorität: **03.09.79 DE 2935547**

(43) Veröffentlichungstag der Anmeldung: **08.04.81 Patentblatt 81/14**

(45) Bekanntmachung des Hinweises auf die Patenterteilung: **25.08.82 Patentblatt 82/34**

(84) Benannte Vertragsstaaten: **AT BE CH DE FR GB IT LI NL**

(56) Entgegenhaltungen:
**DE-A-2 538 790**
**DE-B-2 461 593**

(73) Patentinhaber: **Henkel Kommanditgesellschaft auf Aktien, Postfach 1100 Henkelstrasse 67, D-4000 Düsseldorf 1 (DE)**

(72) Erfinder: **Conrad, Jens, Dr., Dürerweg 15, D-4010 Hilden (DE)**
Erfinder: **Bruns, Klaus, Dr., Notburgaweg 6, D-4150 Krefeld-Traar (DE)**
Erfinder: **Upadek, Horst, Dr., Kempenweg 18 a, D-4006 Erkrath 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 026 323 B1

»4(5)-Acetyl-9,9-dimethyltricyclo-[4.4.0.1^{8,10}]-undec-1-en, dessen Herstellung und Verwendung als Riechstoff, sowie dieses enthaltende Riechstoffkompositionen«

Es wurde gefunden, daß das Isomerengemisch 4(5)-Acetyl-9,9-dimethyltricyclo-[4.4.0.$1^{8,10}$]-undec-1-en einen wertvollen neuen Riechstoff mit einer kräftigen, warmen, holzigen Geruchsnote darstellt.

Die Herstellung des erfindungsgemäßen neuen Isomerengemisches erfolgt nach an sich bekannten Syntheseverfahren der organischen Chemie. Als Ausgangsmaterial für die Synthese dient das käufliche Terpenderivat Nopol. Dieses läßt sich in bekannter Weise, wie dies z. B. von G. Ohloff und G. Schade in der Angewandten Chemie, 67. Jahrgang, 1955, Seite 427 beschrieben wird, zum Nopadien dehydratisieren. Durch Umsetzung von Nopadien mit Methylvinylketon in einer Diels-Alder-Reaktion gelangt man zu dem erfindungsgemäßen Isomerengemisch. Am zweckmäßigsten werden dazu die beiden Ausgangskomponenten Nopadien und Methylvinylketon im Autoklaven mehrere Stunden auf eine Temperatur von ca. 160°C erhitzt. Das nach Fraktionierung erhaltene Produkt stellt ein Gemisch von Strukturisomeren bezüglich der Positionen 4 und 5, beziehungsweise von Stereoisomeren bezüglich der Positionen 5 und 6 dar. Die Synthese erfolgt dabei nach folgendem Reaktionsschema:

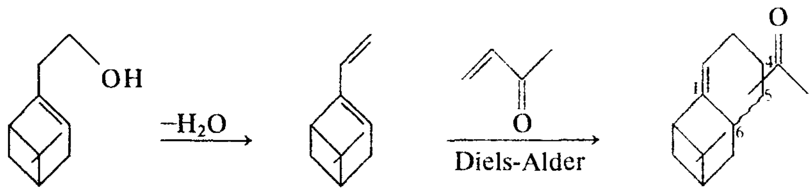


Nopol — Nopadien — 4(5)-Acetyl-9,9-dimethyltricyclo[4.4.0.$1^{8,10}$]undec-1-en

Der neue Riechstoff stellt demnach ein Gemisch folgender Struktur-Isomerer dar:

4-Acetyl-9,9-dimethyltricyclo-[4.4.0.$1^{8,10}$]-undec-1-en,
5-Acetyl-9,9-dimethyltricyclo-[4.4.0.$1^{8,10}$]-undec-1-en.

Das Isomerengemisch 4(5)-Acetyl-9,9-dimethyltricyclo-[4.4.0.$1^{8,10}$]-undec-1-en zeichnet sich durch eine kräftige, warme, holzige Geruchsnote sowie eine gute Haftfestigkeit aus. Ein weiterer Vorteil ist seine sehr gute Kombinationsfähigkeit zu neuartigen Kompositionen, denen es gleichfalls eine gute Haftfestigkeit und interessante Geruchsnuancen verleiht.

Das Isomerengemisch 4(5)-Acetyl-9,9-dimethyltricyclo[4.4.0.$1^{8,10}$]-undec-1-en kann mit anderen Riechstoffen in den verschiedensten Mengenverhältnissen zu neuen Riechstoffkompositionen gemischt werden. Im allgemeinen wird sich sein Anteil in den Riechstoffkompositionen in den Mengen von 1 bis 50 Gewichtsprozent, bezogen auf die gesamte Komposition, bewegen. Derartige Kompositionen können zur Parfümierung von kosmetischen Präparaten wie Cremes, Lotionen, Duftwässern, Aerosolen, Toiletteseifen als auch in der Extrait-Parfümerie verwendet werden. Sie können aber auch zur Geruchsverbesserung technischer Produkte wie Wasch- und Reinigungsmittel, Weichspüler, Textilbehandlungsmittel eingesetzt werden. Zur Parfümierung der verschiedenen Produkte werden diesen die Kompositionen im allgemeinen in Konzentrationen von 0,05 bis 2 Gewichtsprozent, bezogen auf das gesamte Produkt, zugesetzt.

Die nachfolgenden Beispiele sollen den Gegenstand der Erfindung näher erläutern, ohne ihn jedoch hierauf zu beschränken.

## Beispiele

### Herstellung von 4(5)-Acetyl-9,9-dimethyltricyclo-[4.4.0.$1^{8,10}$]-undec-1-en

148,2 g (1 Mol) frisch aus Nopol hergestelltes Nopadien und 70,1 g (1 Mol) Methylvinylketon wurden 6 Stunden lang im Autoklaven unter einem Druck von 5 bar Stickstoff auf 160°C erhitzt. Nach Abdestillieren der Vorläufe wurde das Rohprodukt im Hochvakuum fraktioniert. Es wurden 110 g, das sind 50% der Theorie, an 4(5)-Acetyl-9,9-dimethyltricyclo[4.4.0.$1^{8,10}$]undec-1-en mit folgenden Kennzahlen erhalten:

Siedepunkt 78–80°C bei 0,01 mbar
Brechungsindex $n_D^{20}$ = 1,5132
IR (Film): 1710 $cm^{-1}$ (Keton)
$^1H$-NMR($CCl_4$) : $\delta$ = 5,18 ppm, (m, 1 H, C=CH).

Das Produkt besaß eine kräftige, warme, holzige Geruchsnote.

Beispiel 1

Riechstoffkomposition Holzbase für Herren-Cologne

| | |
|---|---|
| 4(5)-Acetyl-9,9-dimethyltricyclo[4.4.0.$1^{8,10}$]-undec-1-en | 300 Gewichtsteile |
| $\alpha$-Hexylzimtaldehyd | 80 Gewichtsteile |
| Linalylacetat | 80 Gewichtsteile |
| Lyral® (IFF) | 60 Gewichtsteile |
| $\gamma$-Methyljonon | 60 Gewichtsteile |
| Patchouliöl | 50 Gewichtsteile |
| Terpinylacetat | 50 Gewichtsteile |
| Galaxolide® (IFF) | 50 Gewichtsteile |
| Cumarin | 40 Gewichtsteile |
| Amylsalicylat | 40 Gewichtsteile |
| p-tert.-Butyl-cyclohexylacetat | 40 Gewichtsteile |
| Benzylacetat | 30 Gewichtsteile |
| Muskatellersalbeiöl | 30 Gewichtsteile |
| Aldehyd c 12 (10% in DPG) | 30 Gewichtsteile |
| Lemongrasöl | 20 Gewichtsteile |
| Vetiverylacetat | 20 Gewichtsteile |
| Geraniumöl | 10 Gewichtsteile |
| Rosenoxid (10% in DPG) | 10 Gewichtsteile |
| | 1000 Gewichtsteile |

(DPG = Dipropylenglykol).

**Patentansprüche**

1. 4(5)-Acetyl-9,9-dimethyltricyclo[4.4.0.$1^{8,10}$]-undec-1-en.

2. Verfahren zur Herstellung des Isomerengemisches 4(5)-Acetyl-9,9-dimethyltricyclo[4.4.0.$1^{8,10}$]-undec-1-en durch Umsetzung nach Diels-Alder von Nopadien und Methylvinylketon im Autoklaven bei circa 160°C während mehrerer Stunden unter Inertgasatmosphäre und Fraktionierung des Rohproduktes.

3. Verwendung des Isomerengemisches 4(5)-Acetyl-9,9-dimethyltricyclo[4.4.0.$1^{8,10}$]undec-1-en als Riechstoff.

4. Riechstoffkompositionen, dadurch gekennzeichnet, daß sie das Isomerengemisch 4(5)-Acetyl-9,9-dimethyltricyclo[4.4.0.$1^{8,10}$]undec-1-en in einer Menge von 1 – 50 Gewichtsprozent, bezogen auf die gesamte Komposition enthalten.

**Claims**

1. 4(5)-acetyl-9,9-dimethyltricyclo[4.4.0.$1^{8,10}$]undec-1-ene.

2. A process for producing the isomer mixture 4(5)-acetyl-9,9-dimethyltricyclo[4.4.0.$1^{8,10}$]-undec-1-ene by the Diels-Alder reaction of nopadiene and methyl vinyl ketone for several hours in an inert gas atmosphere in an autoclave at around 160°C, followed by fractionation of the crude product obtained.

3. The use of the isomer mixture 4(5)-acetyl-9,9-dimethyltricyclo[4.4.0.$1^{8,10}$]undec-1-ene as a fragrance.

4. Fragrance compositions, characterised in that they contain the isomer mixture 4(5)-acetyl-9,9-dimethyltricyclo[4.4.1.$0^{8,10}$]-undec-1-ene in a quantity of from 1 to 50% by weight, based on the composition as a whole.

**Revendications**

1. Le 4(5)-acétyl-9,9-diméthyltricyclo-[4.4.0.$1^{8,10}$]-undéca-1-ène.

2. Procédé de préparation du mélange d'isomères 4(5)-acétyl-9,9-diméthyltricyclo-[4.4.0.$1^{8,10}$]-undéca-1-ène par réaction de Diels-Alder entre le nopadiène et la méthylvinylcétone à l'autoclave à une température d'environ 160°C pendant plusieurs heures en atmosphère de gaz inerte, et fractionnement du produit brut.

3. Utilisation du mélange d'isomères 4(5)-acétyl-9,9-diméthyltricyclo-[4.4.0.$1^{8,10}$]-undéca-1-ène en tant que matière aromatique.

4. Compositions de parfums caractérisées en ce qu'elles contiennent le mélange d'isomères 4(5)-acétyl-9,9-diméthyltricyclo-[4.4.0.$1^{8,10}$]-undéca-1-ène en quantité de 1 à 50% en poids par rapport à la composition totale.